# EUROPÄISCHE PATENTSCHRIFT

(11) **EP 3 314 491 B1**
(45) Veröffentlichungstag und Bekanntmachung des Hinweises auf die Patenterteilung: **28.12.2022**
(21) Anmeldenummer: 16759967.9
(22) Anmeldetag: 16.06.2016
(51) Int. Cl.: G16H 10/60, G16H 20/40, G16H 30/20

(54) **VERFAHREN ZUR PATIENTENVALIDIERUNG UND VORRICHTUNG ZUR ANWENDUNG DES VERFAHRENS**
METHOD FOR VALIDATING PATIENTS, AND DEVICE FOR USING THE METHOD
PROCÉDÉ DE VALIDATION DE PATIENTS ET DISPOSITIF D'UTILISATION DU PROCÉDÉ

(30) Priorität: 23.06.2015 DE 102015211567
(43) Veröffentlichungstag der Anmeldung: 02.05.2018
(73) Patentinhaber: Opasca GmbH, 68163 Mannheim (DE)
(72) Erfinder: LIEBSCHER, Steffen, 67063 Ludwigshafen (DE); MACHMER, Timo, 67141 Neuhofen (DE); SWERDLOW, Alexej, 67071 Ludwigshafen (DE)
(74) Vertreter: Ullrich & Naumann PartG mbB
(86) Internationale Anmeldenummer: PCT/DE2016/200279
(87) Internationale Veröffentlichungsnummer: WO 2016/206681

(56) Entgegenhaltungen:
- CN-U- 201 788 708
- US-A1- 2011 153 341
- US-A1- 2013 251 099

## Beschreibung

Die Erfindung betrifft ein Verfahren zur EDV-gestützten Patientenvalidierung im Vorfelde der Durchführung einer Strahlentherapie. Des Weiteren betrifft die Erfindung eine Vorrichtung zur Anwendung des erfindungsgemäßen Verfahrens, wobei der Begriff "Vorrichtung" im weitesten Sinne zu verstehen ist. Letztendlich geht es hier um eine komplette Einrichtung, die einzelne Vorrichtungskomponenten umfasst.

Die Strahlentherapie umfasst die Behandlung von gut- und bösartigen Erkrankungen. Sie wird regelmäßig von Fachärzten für Radiologie oder für Strahlentherapie unter Mitwirkung von medizinisch-technischen Assistenten und spezialisierten Medizinphysikern ausgeübt. Dabei wird ionisierende Strahlung auf den Menschen gerichtet, regelmäßig unter Zugrundelegung einer Bestrahlungsplanung aufgrund des komplexen Bestrahlungsprozesses. Eine umfangreiche organisatorische und technische Qualitätssicherung soll dafür sorgen, dass Bestrahlungsfehler weitestgehend auszuschließen sind.

Die Sicherheit in der Strahlentherapie umfasst sowohl die Sicherheit des Fachpersonals als auch die Sicherheit von Patienten, u.a. auch von an der Therapie nicht direkt beteiligten Dritten (z.B. von Begleitpersonen oder Krankentransporteuren). Automatisierte Abläufe und redundante Sicherheitssysteme sollen dem Sicherheitsbedürfnis entsprechen. Ein ganzheitlicher Lösungsansatz ist hier von Vorteil, wobei eine patientenspezifische bzw. patientenindividuelle Ausrichtung angestrebt wird.

Zu den aktiven Sicherheitsvorkehrungen gehört die Patientenvalidierung, wonach die Bestrahlung des falschen Patienten bzw. die Verwechslung von Patienten vermieden werden soll. Dabei geht es darum, den Patienten eindeutig zu identifizieren und ihm erst nach einer erfolgreichen Patientenvalidierung der Behandlung zuzuführen bzw. die Behandlung erst nach eindeutiger Sicherstellung der Patientenidentität zu starten.

Aus der Praxis ist es bereits bekannt, dass patientenspezifische Daten erfasst und gespeichert werden. Eine Patientenvalidierung wird nur insoweit durchgeführt, als der Patient befragt wird und allenfalls visuelle Vergleiche, beispielsweise anhand von fotografischen Abbildungen, vorgenommen werden. Verwechslungen im hektischen Klinik-/Behandlungsbetrieb sind nicht auszuschließen.

Aus der US 2013/0251099 A1 ist ein Verfahren zur Patientenidentifizierung bekannt, wobei zur Patientenidentifikation anatomische, physiologische und funktionelle Informationen über Gewebe und Organe mittels medizinischer Bilddaten herangezogen werden, die im Rahmen der Behandlung durch Röntgen-Radiographie, Computertomografie, Magnetresonanztomografie, Ultraschall u.ä. aufgenommen werden.

Aus der US 2011/0153341 A1 ist ein Patientenidentifikationssystem und ein Verfahren zur Patientenidentifikation bekannt, um die Identifikation eines Patienten im Klinikalltag zu erleichtern. Hierfür werden Fotografien von Patienten aufgenommen und mit weiteren Patienteninformationen verknüpft und durch ein einfaches User-Interface durch das Klinikpersonal abgeglichen.

Aus der CN 201788708 U ist eine Sicherheitssteuervorrichtung für Bestrahlungskammern bekannt, umfassend ein Verriegelungssystem und ein Fingerabdruckleser. Mit Hilfe eines Fingerabdruck-Identifizierungssystems kann die Sicherheitssteuereinrichtung wirksam verhindern, dass nicht berechtigte Mitarbeiter in eine Bestrahlungskammer eintreten und versehentlich eine Bestrahlungsvorrichtung starten.

Der vorliegenden Erfindung liegt daher die Aufgabe zugrunde, ein Verfahren zur Patientenvalidierung anzugeben, wonach die Behandlung bzw. Bestrahlung falscher Patienten bzw. die Verwechslung von Patienten im Vorfeld einer Behandlung weitestgehend ausgeschlossen ist. Des Weiteren soll eine entsprechende Vorrichtung zur Anwendung des erfindungsgemäßen Verfahrens angegeben werden.

Voranstehende Aufgabe wird durch die Merkmale der nebengeordneten Ansprüche 1 und 6 gelöst. Danach werden patientenspezifische Daten erfasst und in einer Validierungsdatenbank als Referenzmerkmale für den jeweiligen Patienten abgelegt. Vor jeder Behandlung werden am Patienten aktuelle Patientenmerkmale ermittelt und werden diese mit den Referenzmerkmalen aus der Validierungsdatenbank verglichen. Eine Freigabe zur Behandlung erfolgt nur dann, wenn der über Computer und dort zum Einsatz kommende Algorithmen vorgenommene Vergleich ein vorgegebenes Maß an Übereinstimmung ergibt. Als patientenspezifische Daten dienen biometrische Charakteristika des Gesichts, wobei die biometrischen Charakteristika sowohl zur Generierung der Referenzmerkmale als auch zur Ermittlung der aktuellen Patientenmerkmale kamerabasiert ermittelt und in einem Rechner verarbeitet werden und wobei die kamerabasierte Ermittlung im Eingangsbereich eines Bestrahlungsraums vorgesehen ist, wobei die Patientenvalidierung automatisch durch das Betreten eines Behandlungsraumes oder durch das Schließen eines Strahlenschutztores ausgelöst wird, und wobei schließlich automatisch in Abhängigkeit von der Patientenvalidierung eine Freigabe oder ein Abbruch der Operation erfolgt.

Die erfindungsgemäße Vorrichtung umfasst einen Rechner, der auf eine ggf. externe Datenbank mit Daten zur Patientenidentifizierung zugreift, wobei mittels dieser Daten auf eine zweite Datenbank, eine Validierungsdatenbank, zugegriffen wird, die patientenspezifische Daten enthält, wobei aktuelle spezifische Daten des Patienten mit Referenzmerkmalen aus der Validierungsdatenbank verglichen werden und wobei sowohl die Referenzmerkmale als auch die aktuellen Patientenmerkmale mittels Kamerasystemen ermittelt werden, wobei als patientenspezifische Daten biometrische Charakteristika des Gesichts dienen, wobei das Kamerasystem im Eingangsbereich eines Bestrahlungsraums vorgesehen ist, wobei die Patientenvalidierung automatisch durch das Betreten eines Behandlungsraumes oder durch das Schließen eines Strahlenschutztores ausgelöst wird, und wobei schließlich automatisch in Abhängigkeit von der Patientenvalidierung eine Freigabe oder ein Abbruch der Operation erfolgt.

An dieser Stelle sei angemerkt, dass es hier um eine EDV-gestützte Patientenvalidierung geht, unter Nutzung einer Peripherie zur Erfassung patientenspezifischer Daten und eines Rechners, der die patientenspezifischen Daten aufbereitet und einer Validierungsdatenbank zuführt. Von dort sind die patientenspezifischen Daten zur vergleichenden Betrachtung, insbesondere zum Vergleich mittels Algorithmen, abrufbar, nämlich über eine Patientenidentifizierung, die auf einer separaten Datenbank, beispielsweise einer externen Datenbank, abgelegt sein kann. Die Patentenidentifizierung kann beispielsweise den Namen des Patienten und die bibliografischen Daten enthalten, über die der Patient eindeutig definierbar ist.

Es werden über die Patienten-ID Referenzmerkmale aus der Validierungsdatenbank abgerufen und werden diese mit aktuellen Merkmalen der zu behandelnden Person verglichen, wobei dieser Vergleich auch "manuell" von einer Bedienperson durchführbar ist, nämlich bei entsprechender Darstellung der Referenzmerkmale auf einem Monitor.

Wie bereits zuvor ausgeführt, dienen die patientenspezifischen Daten in der Validierungsdatenbank zur eindeutigen Patientenidentifizierung, wobei eine entsprechende Patienten-ID zum Abrufen der patientenspezifischen Daten aus der Validierungsdatenbank dient. Auf diese Weise lassen sich die Daten zusammenführen.

In weiter vorteilhafter Weise gehören zu den patientenspezifischen Daten fotografische Aufnahmen bzw. Kamerabilder des Patienten.

Die so gewonnenen Daten werden in einem Rechner verarbeitet und beispielsweise der Referenzdatenbank bzw. dem rechnerischen Vergleich per Algorithmus zugeführt.

Gerade über die Zeitspanne der Strahlentherapie hinweg verändert sich das Aussehen des Patienten. Daher ist es von Vorteil, dass über die Phase der Behandlung hinweg die Referenzmerkmale an veränderte Patientenmerkmale angepasst werden bzw. dass die Referenzmerkmale zur Anpassung an die Realität nachgeführt werden. Dabei ist sicherzustellen, dass es sich beim Nachführen der Referenzmerkmale tatsächlich um Merkmale desselben Patienten handelt.

Im Rahmen einer automatischen Abwicklung der Strahlenbehandlung ist es von weiterem Vorteil, wenn die Freigabe zur Behandlung unmittelbar oder mittelbar auf das Behandlungsgerät und/oder die Behandlungstherapie wirkt, nämlich nach positiver Patientenvalidierung. Es erfolgt danach eine Interlockfreigabe, so dass der Bestrahlungsvorgang bei ordnungsgemäß positioniertem Patienten beginnen kann.

Es muss nicht nur ausgeschlossen werden, dass der falsche Patient behandelt wird. Vielmehr gilt es auch zu vermeiden, dass sich ein Patient mit falscher Identität beispielsweise im Gefahrenbereich eines Bestrahlungsraums befindet.

So ist es beispielsweise denkbar, dass am Eingangsbereich eines Bestrahlungsraums ein Tor-Terminal vorgesehen ist, von dem aus bei Feststellung eines Patienten mit falscher Identität im Gefahrenbereich eine Identitätsermittlung, eine Analyse anhand der patientenspezifischen Daten/Referenzmerkmale und schließlich eine Freigabe oder ein Abbruch der Operation erfolgen kann. Insoweit kommt der Vorkehrung eines Tor-Terminals im Eingangsbereich des Behandlungs-/Bestrahlungsraums eine ganz besondere Bedeutung zu.

Es gibt nun verschiedene Möglichkeiten, die Lehre der vorliegenden Erfindung in vorteilhafter Weise auszugestalten und weiterzubilden. Dazu ist einerseits auf die dem Anspruch 1 nachgeordneten Ansprüche und andererseits auf die nachfolgende Erläuterung eines bevorzugten Ausführungsbeispiels der Erfindung anhand der Zeichnung zu verweisen. In Verbindung mit der Erläuterung des bevorzugten Ausführungsbeispiels der Erfindung anhand der Zeichnung werden auch im Allgemeinen bevorzugte Ausgestaltungen und Weiterbildungen der Lehre erläutert. In der Zeichnung zeigen
- Fig. 1: anhand einer Grafik den grundsätzlichen Ablauf des erfindungsgemäßen Verfahrens und
- Fig. 2: anhand eines Ablaufdiagramms, wie das erfindungsgemäße Verfahren im Detail ablaufen kann.

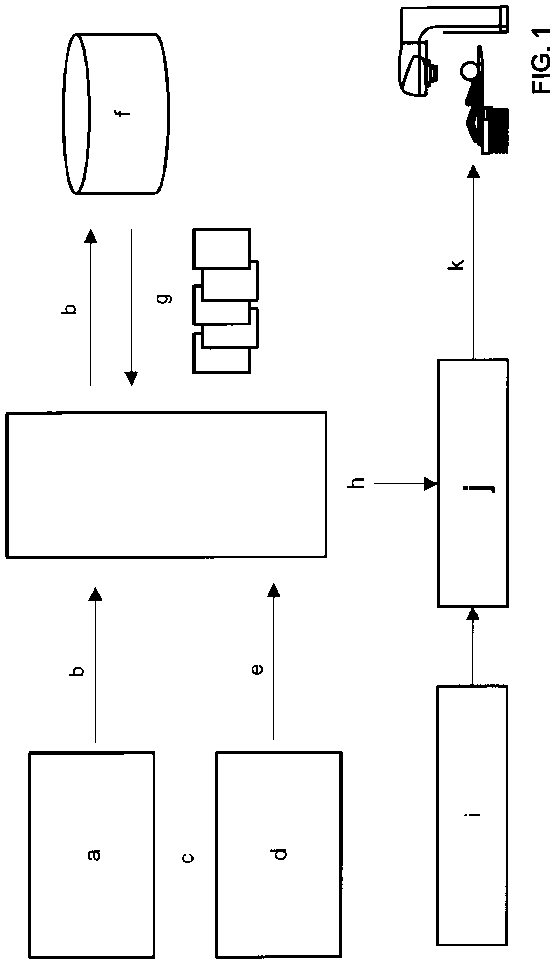

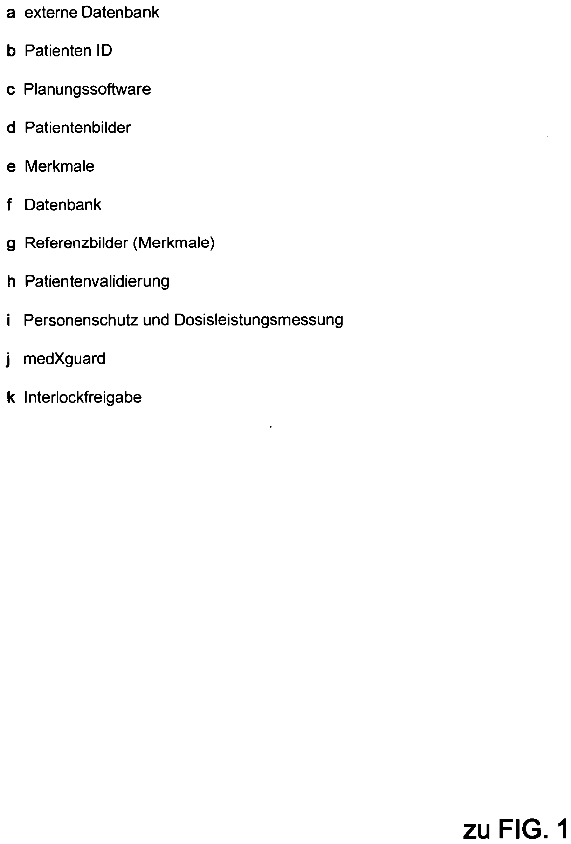

Fig. 1 zeigt den grundsätzlichen Ablauf des erfindungsgemäßen Verfahrens, wonach eine Patienten-ID in einer möglicherweise externen Datenbank abgelegt ist. Über den Namen des Patienten, Geburtsdatum, etc. kann die Patienten-ID eindeutig gezogen werden, wobei diese zum Zugriff auf Referenzmerkmale aus einer Validierungsdatenbank dient.

Zu den Referenzmerkmalen gehören biometrischen Charakteristika, aber auch eine oder mehrere fotografische Abbildungen des Patienten. So lassen sich über die Patienten-ID eindeutig Referenzmerkmale aus der Validierungsdatenbank ziehen, die mit aktuellen Patientenmerkmalen des zur Behandlung anstehenden Patienten vorzugsweise automatisch verglichen werden, nämlich im Rahmen der EDV-gestützten Patientenvalidierung. Der Vergleich fotografischer Bilder wie auch der Vergleich biometrischer Charakteristika ist in Kombination möglich, wobei ein automatischer EDV-Vergleich unter Zugrundelegung geeigneter Algorithmen erfolgen kann.

Nur nach erfolgreicher Patientenvalidierung, d.h. nach eindeutiger Bestätigung des "richtigen" Patienten, erfolgt eine Interlockfreigabe mit ärztlich vorgegebener Dosisleistung am Gerät.

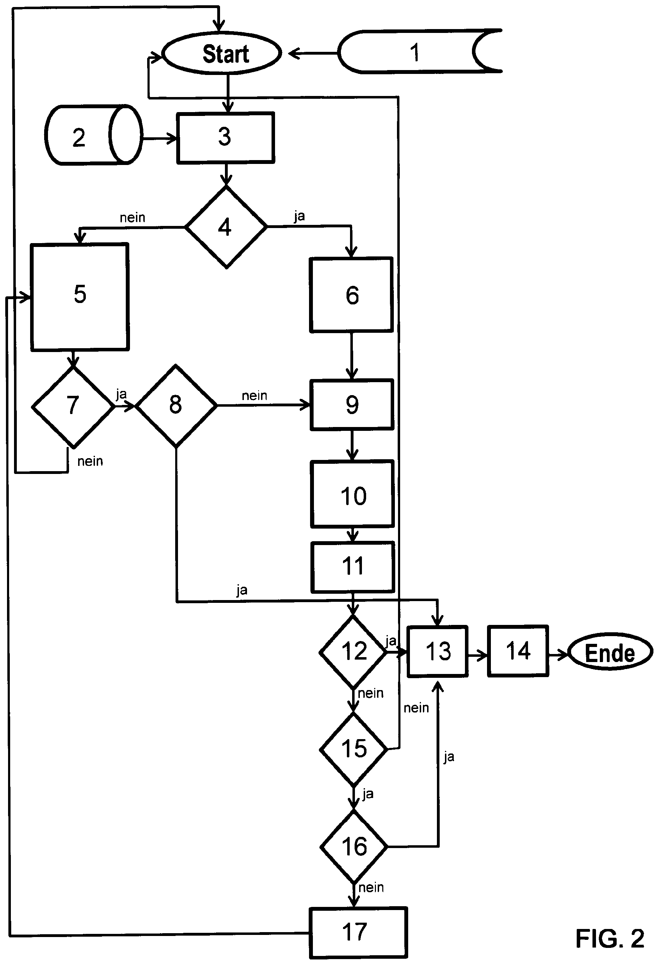

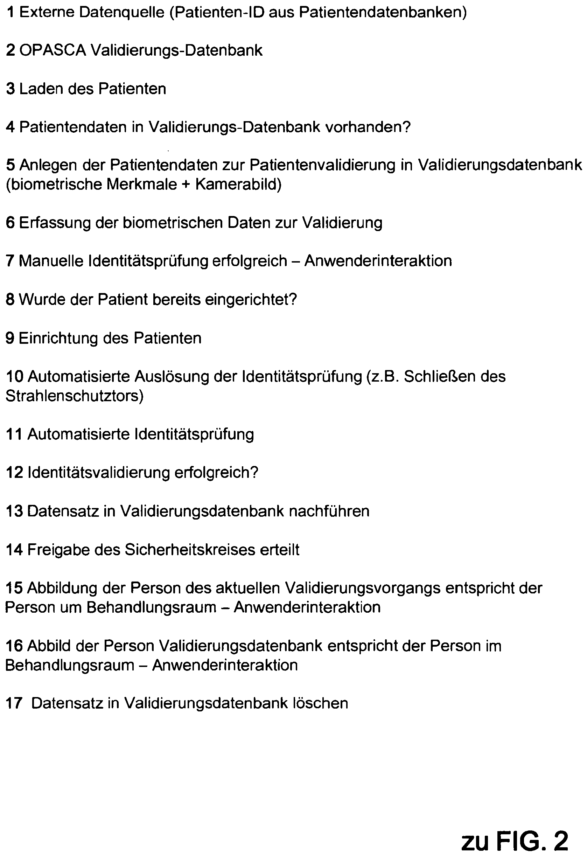

Fig. 2 zeigt anhand eines Ablaufdiagramms, wie die Patientenvalidierung im Sinne eines automatischen Ablaufs erfolgen kann, bis hin zur Freigabe des Sicherheitskreises, wonach der Bestrahlungsvorgang erfolgen kann. Da das Ablaufdiagramm mit der einbeschriebenen Erklärung selbstredend ist, erübrigen sich weitere Ausführungen dazu unter Hinweis auf die voranstehenden Ausführungen zu Fig. 1.

Im Rahmen des erfindungsgemäßen Verfahrens ist wesentlich, dass unter Nutzung einer geeigneten Peripherie, umfassend Kameras und Kamerasysteme biometrische Charakteristika ermittelt werden, sowohl zur Erzeugung der Patientendaten für die Validierungsdatenbank als auch zur Ermittlung aktueller Patientenmerkmale zum Vergleich mit den hinterlegten Referenzmerkmalen.

Es ist denkbar, bei der Validierung beliebige Redundanzen und Sicherheitskriterien einzubauen, bis hin zur einer alternativen oder abschließenden Beurteilung durch eine Bedienperson, beispielsweise an einem Eingangsterminal, am Schreibtisch eines zugeordneten Bearbeitungsraumes, etc. Ein erhebliches Maß an Sicherheit kann geschaffen werden, und dies zum Wohle der zu behandelnden Patienten, indem nämlich wirksam die Verwechslung von Patienten in Bezug auf eine ärztlich definierte bzw. vorgegebene Bestrahlung so gut wie ausgeschlossen werden kann.

Hinsichtlich weiterer vorteilhafter Ausgestaltungen des erfindungsgemäßen Verfahrens wird zur Vermeidung von Wiederholungen auf den allgemeinen Teil der Beschreibung sowie auf die beigefügten Ansprüche verwiesen.

Schließlich sei ausdrücklich darauf hingewiesen, dass das voranstehend beschriebene Ausführungsbeispiel des erfindungsgemäßen Verfahrens lediglich zur Erörterung der beanspruchten Lehre dient, diese jedoch nicht auf das Ausführungsbeispiel einschränkt.

## Patentansprüche

1. Verfahren zur EDV-gestützten Patientenvalidierung im Vorfelde der Durchführung einer Strahlentherapie, wobei patientenspezifische Daten erfasst und in einer Validierungsdatenbank als Referenzmerkmale hinterlegt werden, wobei vor jeder Behandlung aktuelle Patientenmerkmale ermittelt und mit den Referenzmerkmalen verglichen werden und wobei eine Freigabe zur Behandlung nur dann erfolgt, wenn der Vergleich ein vorgegebenes Maß an Übereinstimmung ergibt, **dadurch gekennzeichnet, dass**
als patientenspezifische Daten biometrische Charakteristika des Gesichts dienen, wobei die biometrischen Charakteristika sowohl zur Generierung der Referenzmerkmale als auch zur Ermittlung der aktuellen Patientenmerkmale kamerabasiert ermittelt und in einem Rechner verarbeitet werden und
wobei die kamerabasierte Ermittlung im Eingangsbereich eines Bestrahlungsraums vorgesehen ist,
wobei die Patientenvalidierung automatisch durch das Betreten eines Behandlungsraumes oder durch das Schließen eines Strahlenschutztores ausgelöst wird, und
wobei schließlich automatisch in Abhängigkeit von der Patientenvalidierung eine Freigabe oder ein Abbruch der Operation erfolgt.

2. Verfahren nach Anspruch 1, **dadurch gekennzeichnet, dass** die patientenspezifischen Daten der Validierungsdatenbank über eine Patientenidentifizierung, vorzugsweise aus einer externen Patientendatenbank, adressierbar bzw. abrufbar sind.

3. Verfahren nach Anspruch 1 oder 2, **dadurch gekennzeichnet, dass** die patientenspezifischen Daten eine fotografische Aufnahme bzw. ein Kamerabild des Patienten umfassen.

4. Verfahren nach einem der Ansprüche 1 bis 3, **dadurch gekennzeichnet, dass** über die Phase der Behandlungen hinweg die Referenzmerkmale an veränderte Patientenmerkmale angepasst bzw. die Referenzmerkmale zur Anpassung an die Realität nachgeführt werden.

5. Verfahren nach einem der Ansprüche 1 bis 4, **dadurch gekennzeichnet, dass** die Freigabe zur Behandlung unmittelbar oder mittelbar auf das Behandlungsgerät und/oder die Behandlungsperipherie wirkt.

6. Vorrichtung zur Anwendung eines Verfahrens nach einem der Ansprüche 1 bis 5, mit einem Rechner, der auf eine ggf. externe Datenbank mit Daten zur Patientenidentifizierung zugreift, wobei mittels dieser Daten auf eine zweite Datenbank, eine Validierungsdatenbank, zugegriffen wird, die patientenspezifische Daten enthält, wobei aktuelle spezifische Daten der Patienten mit Referenzmerkmalen aus der Validierungsdatenbank verglichen werden**dadurch gekennzeichnet, dass**
sowohl die Referenzmerkmale als auch die aktuellen Patientenmerkmale mittels Kamerasystemen ermittelt werden, wobei als patientenspezifische Daten biometrische Charakteristika des Gesichts dienen, wobei das Kamerasystem im Eingangsbereich eines Bestrahlungsraums vorgesehen ist,
wobei die Patientenvalidierung automatisch durch das Betreten eines Behandlungsraumes oder durch das Schließen eines Strahlenschutztores ausgelöst wird,
und wobei schließlich automatisch in Abhängigkeit von der Patientenvalidierung eine Freigabe oder ein Abbruch der Operation erfolgt.

## Claims

1. Method for EDP-based patient validation before carrying out radiotherapy, wherein patient-specific data are detected and stored in a validation database as reference features, wherein, prior to each treatment, current patient features are established and compared with the reference features and wherein a release for treatment is carried out only when the comparison produces a predetermined degree of correspondence, **characterised in that**
biometric characteristics of the face are used as patient-specific data, wherein the biometric characteristics both for generating the reference features and for establishing the current patient features are established on the basis of a camera and processed in a computer, and
wherein the camera-based establishment is provided in the entrance region of a radiation room,
wherein the patient validation is automatically initiated by entering a treatment room or by closing a radiation protection door, and
wherein finally a release or a termination of the operation is automatically carried out depending on the patient validation.

2. Method according to claim 1, **characterised in that** the patient-specific data of the validation database can be addressed or called up via a patient identification, preferably from an external patient database.

3. Method according to claim 1 or 2, **characterised in that** the patient-specific data items include a photographic recording or camera image of the patient.

4. Method according to any one of claims 1 to 3, **characterised in that** in the course of the treatments the reference features are adapted to changed patient features or the reference features are updated to adapt to reality.

5. Method according to any one of claims 1 to 4, **characterised in that** the release for treatment acts directly or indirectly on the treatment device and/or the peripheral treatment equipment.

6. Apparatus for applying a method according to any one of claims 1 to 5, having a computer, which has access to a where applicable external database with data items for patient identification, wherein by means of these data items access is afforded to a second database, a validation database, which contains patient-specific data, wherein current specific data of the patients are compared with reference features from the validation database, **characterised in that** both the reference features and the current patient features are established by means of camera systems, wherein biometric characteristics of the face are used as patient-specific data, wherein the camera system is provided in the entrance region of a radiation room, wherein the patient validation is automatically initiated by entering a treatment room or by closing a radiation protection door,
and wherein finally a release or a termination of the operation is automatically carried out depending on the patient validation.

## Revendications

1. Procédé de validation de patient assistée par ordinateur préalablement à la réalisation d'une radiothérapie, des données spécifiques au patient étant saisies et enregistrées en tant que caractéristiques de référence dans une base de données de validation, des caractéristiques de patient actuelles étant déterminées avant tout traitement et comparées aux caractéristiques de référence, et une autorisation de traitement n'intervenant que si la comparaison fournit une quantité prédéfinie de coïncidence, **caractérisé en ce que**,
en tant que données spécifiques au patient, on utilise des caractéristiques biométriques du visage, les caractéristiques biométriques étant déterminées sur la base d'une caméra aussi bien pour la production des caractéristiques de référence que pour la détermination des caractéristiques de patient actuelles, et étant traitées dans un ordinateur, et la détermination sur la base d'une caméra étant prévue dans la zone d'entrée d'une chambre d'irradiation,
la validation de patient étant déclenchée automatiquement par la pénétration dans une chambre de traitement ou par la fermeture d'une porte de protection contre les rayons, et une autorisation ou une interruption de l'opération survenant finalement automatiquement en fonction de la validation de patient.

2. Procédé selon la revendication 1, **caractérisé en ce que** les données spécifiques au patient de la base de données de validation peuvent être adressées ou respectivement appelées par le biais d'une identification du patient, de préférence à partir d'une base de données de patient externe.

3. Procédé selon la revendication 1 ou 2, **caractérisé en ce que** les données spécifiques au patient comprennent une prise de vue photographique ou respectivement une image de caméra du patient.

4. Procédé selon l'une des revendications 1 à 3, **caractérisé en ce que**, pendant la phase des traitements, les caractéristiques de référence sont adaptées à des caractéristiques de patient modifiées ou respectivement les caractéristiques de référence sont actualisées pour être adaptées à la réalité.

5. Procédé selon l'une des revendications 1 à 4, **caractérisé en ce que** l'autorisation de traitement agit directement ou indirectement sur l'appareil de traitement et/ou la périphérie de traitement.

6. Dispositif d'utilisation d'un procédé selon l'une des revendications 1 à 5, avec un ordinateur qui accède à une base de données éventuellement extérieure contenant des données d'identification de patient, un accès étant, au moyen de ces données, réalisé à une deuxième base de données, une base de données de validation, qui contient des données spécifiques au patient, des données spécifiques actuelles des patients étant comparées à des caractéristiques de référence issues de la base de données de validation, **caractérisé en ce que**
aussi bien les caractéristiques de référence que les caractéristiques de patient actuelles sont déterminées au moyen de systèmes de caméra, des caractéristiques biométriques du visage servant de données spécifiques au patient, le système de caméra étant prévu dans la zone d'entrée d'une chambre d'irradiation, la validation de patient étant déclenchée automatiquement par la pénétration dans une chambre de traitement ou par la fermeture d'une porte de protection contre les rayons,
et une autorisation ou une interruption de l'opération survenant finalement automatiquement en fonction de la validation de patient.
